# EUROPÄISCHE PATENTSCHRIFT

(11) **EP 2 114 561 B1**
(45) Veröffentlichungstag und Bekanntmachung des Hinweises auf die Patenterteilung: **02.11.2016**
(21) Anmeldenummer: 07857691.5
(22) Anmeldetag: 17.12.2007
(51) Int. Cl.: B01J 21/16, B01J 23/30, B01J 23/652, B01J 23/888, B01J 29/076, B01J 35/00, C07C 45/52, C07C 319/14, B01J 37/04, B01J 23/90, B01J 21/20

(54) **VERFAHREN ZUR DEHYDRATISIERUNG VON GLYCERIN in Anwesenheit von WOLFRAMVERBINDUNGEN ENTHALTENDEn KATALYSATOREN**
METHOD FOR DEHYDRATING GLYCERINE in presence of CATALYSTS CONTAINING TUNGSTEN COMPOUNDS
PROCÉDÉ DE DÉSHYDRATATION DE GLYCÉRINE en présence de CATALYSEURS CONTENANT DES COMPOSÉS DE TUNGSTÈNE

(30) Priorität: 29.01.2007 DE 102007004351
(43) Veröffentlichungstag der Anmeldung: 11.11.2009
(73) Patentinhaber: Evonik Degussa GmbH, 45128 Essen (DE)
(72) Erfinder: REDLINGSHÖFER, Hubert, 91481 Münchsteinach (DE); WECKBECKER, Christoph, 63584 Gründau-Lieblos (DE); HUTHMACHER, Klaus, 63571 Gelnhausen (DE); DÖRFLEIN, Andreas, 63538 Grosskrotzenburg (DE)
(86) Internationale Anmeldenummer: PCT/EP2007/064060
(87) Internationale Veröffentlichungsnummer: WO 2008/092533

(56) Entgegenhaltungen:
- EP-A- 0 598 229
- EP-A- 1 402 947
- WO-A-2006/087083
- US-A- 4 508 918
- US-A- 5 382 731
- US-A1- 2006 183 945
- KUROSAKA ET AL: "Production of 1,3-propanediol by hydrogenolysis of glycerol catalyzed by Pt/WO3/ZrO2" CATALYSIS COMMUNICATIONS, ELSEVIER SCIENCE, AMSTERDAM, NL, Bd. 9, Nr. 6, 4. Dezember 2007 (2007-12-04), Seiten 1360-1363, XP022490054 ISSN: 1566-7367
- TSUKUDA ET AL: "Production of acrolein from glycerol over silica-supported heteropoly acids" CATALYSIS COMMUNICATIONS, ELSEVIER SCIENCE, AMSTERDAM, NL, Bd. 8, Nr. 9, 12. Dezember 2006 (2006-12-12), Seiten 1349-1353, XP022162877 ISSN: 1566-7367

## Beschreibung

Die Erfindung betrifft ein Verfahren zur Herstellung von Acrolein aus Glycerin unter Verwendung eines sauren Katalysators, der wolframhaltige Verbindungen und mindestens einen weiteren Promoter aufweist.

Acrolein ist ein wichtiges Zwischenprodukt und von großer wirtschaftlicher Bedeutung für die Darstellung von Acrylsäure, D,L-Methionin und dem Methionin-Hydroxy-Analogen 2-Hydroxy-4-methylthiobuttersäure (MHA). Methionin ist eine essentielle Aminosäure, die u.a. als Ergänzung in Futtermitteln eingesetzt wird. Nutritivitätsverbessernde Futtermittelzusatzstoffe sind heute ein unverzichtbarer Bestandteil der Tierernährung. Sie dienen der besseren Verwertung des Nahrungsangebotes, stimulieren das Wachstum und fördern die Eiweißbildung. Einer der wichtigsten dieser Zusatzstoffe ist die essentielle Aminosäure Methionin, die vor allem in der Geflügelaufzucht als Futtermitteladditiv eine herausragende Stellung einnimmt. Auf diesem Gebiet haben, aber auch sogenannte Methionin-Ersatzstoffe wie das Methionin-Hydroxy-Analog (abgekürzt MHA) nicht unerhebliche Bedeutung, da sie ähnliche wachstumsstimulierende Eigenschaften aufweisen wie die dafür bekannte Aminosäure. Acrylsäure ist ein wichtiger Ausgangsstoff zur Herstellung von Polymeren, die beispielsweise aufgrund ihres Wasseraufnahmevermögens als Superabsorber eingesetzt werden.

Nach dem Stand der Technik erfolgt die Synthese von Acrolein durch heterogen katalysierte selektive Oxidation von Propen an Mischoxidkatalysatoren. EP 417723 beschreibt die Synthese an komplexen Multimetallmischoxidkatalysatoren bei Temperaturen von 300 bis 380 °C und Drücken von 1,4 bis 2,2 bar. In Ullmann's Encyclopedia of Industrial Chemistry, 6. Auflage, 1999 ist das gesamte Verfahren inklusive Aufarbeitung, bei dem mehrere Nebenprodukte abgetrennt werden, beschrieben. Nachdem das Eduktgemisch aus Propen, Luft und Wasser zumindest teilweise am Katalysator umgesetzt wurde, erfolgt zunächst das Quenchen zur Abtrennung hochsiedender Nebenprodukte wie Polymere, Acrylsäure und Essigsäure. Im anschließenden Absorber wird Acrolein ausgewaschen. Nach der Desorption zur Rückgewinnung des Absorptionsmittels wird das erhaltene Rohacrolein mehrstufig destillativ gereinigt.

Es ist bekannt, dass sich Glycerin in Gegenwart saurer Stoffe zu verschiedenen Produkten dehydratisieren lässt. Gemäß Organic Synthesis I, 15-18 (1964) wird durch Behandeln eines Gemisches aus pulverförmigem Kaliumhydrogensulfat, Kaliumsulfat und Glycerin bei 190 bis 200 °C Acrolein in einer Ausbeute zwischen 33 und 48 % gewonnen. Aufgrund der niedrigen Ausbeuten und der hohen Salzfrachten eignet sich dieses Verfahren jedoch nicht für den technischen Maßstab.

Im Rahmen der Untersuchungen von Modellsubstanzen von Biomasse Pyrolyseölen wurde auch die katalytische Behandlung von Glycerin an H-ZSM5-Zeolithen bei 350 bis 500 °C untersucht - siehe Dao, Le H. et al. ACS Symp. Ser.: 376 (Pyrolysis Oils Biomass) 328-341 (1988). Kohlenwasserstoffe werden nur in geringen Ausbeuten gebildet.

In der EP 0598229, US 5387720 ist ferner die säurekatalysierte Umsetzung von Glycerin zu Acrolein in der Gas- und in der Flüssigphase beschrieben. Darin bestimmt alleine die Säurestärke (Hammet'sche Säurefunktion) die Eignung als Katalysator. Die DE 42 38 492 betrifft die Synthese von 1,2- und 1,3-Propandiol durch Dehydratisierung von Glycerin mit hohen Ausbeuten.

Aus der WO 2006/087083 ist ein Verfahren zur Herstellung von Acrolein aus Glycerin an sauren Katalysatoren bekannt, bei dem man dem Reaktionsgemisch Sauerstoff zusetzt.

Ein ähnliches Verfahren wird in der WO 2006/087084 beschrieben. Die dort eingesetzten Katalysatoren weisen eine Hammett-Acidität H₀ im Bereich von -9 bis -18 auf.

Geeignete Katalysatoren für die Dehydratisierung von Glycerin sind Wolframverbindungen enthaltende Festkörperkatalysatoren mit einer Hammett-Acidität H₀ von < +2, die einen oder mehrere Promotoren enthalten, ausgewählt aus Verbindungen der Gruppe von Elementen, enthaltend, insbesondere bestehend aus Gold, Silber, Kupfer, Platin, Rhodium, Palladium, Ruthenium, Samarium, Cer, Yttrium, Scandium, Lanthan, Zink, Magnesium, Eisen, Kobalt oder Nickel oder deren Mischungen. Als Promotor sind auch insbesondere saure Zeolithe oder Montmorillonit geeignet, die dann gegebenenfalls jeweils in einer Menge von 0,1 bis 30 Gew.-%, insbesondere 5 bis 25 Gew.-%, bezogen auf den Katalysator, zusätzlich zu den oben genannten Promotoren oder allein vorliegen.

In Abhängigkeit von ihrem Normalpotential können einzelne der genannten Elemente auch in metallischer Form auf dem Katalysator vorliegen.

Bevorzugt sind Katalysatoren, die eine Hammett-Acidität H₀ von < +2 bis -20 aufweisen.

Es wurde gefunden, dass die Anwesenheit von Brönsted-Säurezentren an der Katalysatoroberfläche nicht ausreicht, um eine gute Ausbeuten an Acrolein zu erzielen. Zusätzlich wurde gefunden, dass die Gegenwart von Wolframverbindungen und deren Kombination mit sauren Zentren die Ausbeuten verbessert.

Wolframoxid und Palladium enthaltende Festkörperkatalysatoren sind aus der europäischen Offenlegungsschrift 1 402 947 A1 und dem US-Patent 4,508,918 bekannt. Ferner offenbart das US-Patent 5,382,731 einen auf Wolframoxid basierenden Festkörperkatalysator, der zusätzlich zum Wolframoxid auch noch Platin enthält. Allerdings betreffen diese Dokumente nicht die Dehydratisierung von Glycerin zu Acrolein. Vielmehr betreffen die EP 1 402 947 A1 und die US 5,382,731 die Isomerisierung von Paraffin, und die US 4,508,918 betrifft die Hydrierung von aromatischen Kohlenwasserstoffen zu Cyclohexan-Derivaten.

Die Eignung von Festkörperkatalysatoren, die durch Imprägnierung von Silica-Partikeln unterschiedlicher Porengröße mit Siliziumwolframsäure erhalten werden, in der Dehydratisierung von Glycerin zu Acrolein wird in Tsukuda et al. ("Production of acrolein from glycerol over silica-supported heteropolyacids", Catalysis Communication 2007, 8, 1349-1353) offenbart.

Da Glycerin ein reaktives Molekül darstellt, das speziell bei hohen Temperaturen zur Bildung von höhersiedenden Verbindungen neigt, indem zwei oder mehrere Glycerinmoleküle miteinander reagieren, wird der Katalysator durch Ablagerungen von kohlenstoffhaltiger Molekühle auf der Oberfläche verkokt. Dies führt zur Aktivitätsverminderung.

Zur Erzielung einer hohen Raum-Zeit-Ausbeute sind nicht alleine die Säurestärke des Katalysators nach Hammett sondern auch die Regenerierbarkeit und die Neigung zur Verkokung wichtig.

Die Regenerierbarkeit von sauren Festkörperkörperkatalysatoren wird in der Parallelanmeldung EP 07 857 710 (Offenlegungsschrift EP 2 114 564 A1) behandelt. Dieses Dokument offenbart, dass Wolfram enthaltende Festkörperkatalysatoren mit einer Hammett-Acidität H₀ von <+2 dann regenerierbar sind, wenn sie zusätzlich einen Promotor ausgewählt aus der Verbindungen der Gruppe Gold, Silber, Kupfer, Vanadium, Platin, Palladium, Rhodium, Ruthenium, Samarium, Cer, Yttrium, Scandium, Lanthan, Zink, Magnesium, Eisen, Kobalt oder Nickel oder deren Mischungen, und gegebenenfalls zusätzlichen Verbindungen der Elemente Lithium, Natrium, Kalium oder Cäsium und/oder Montmorillonit oder sauren Zeolithen enthalten. Gemäß der Lehre dieses Dokument können diese Katalysatoren, wenn sie nach dem Einsatz in der Dehydratisierung von Glycerin zu Acrolein eine geringere Aktivität und/oder Selektivität als vor ihrem Einsatz aufweisen, regeneriert werden, indem sie einer reduzierenden oder einer oxidierenden Atmosphäre ausgesetzt werden.

Aufgabe der Erfindung ist es, ein Verfahren zur Dehydratisierung von Glycerin zur Verfügung zu stellen, bei welchem der Katalysator eine geringe Verkokungsneigung zeigt und leicht regenerierbar ist.

Erfindungsgemäß wird diese Aufgabe gelöst, indem man Katalysatoren mit Palladium als Promotor einsetzt, der die Regeneration der Katalysatoren beschleunigt. Zusätzlich nehmen Standzeit und Raum-Zeit-Ausbeute deutlich zu, da vor allem die Desaktivierung durch Verkokung bei diesen Katalysatoren zumindest größtenteils beseitigt und die Aktivität deutlich erhöht werden. Somit kann der Umsatz an Glycerin und die Ausbeute zu Acrolein in Abhängigkeit von der Zeit auf hohem Niveau aufrechterhalten werden. Dies ist speziell für eine technische Umsetzung der Synthese von großer Bedeutung, da ein Wechsel des Katalysators und damit verbundene Anlagenstillstände hohe Kosten verursachen. Neben den Brönsted-Säuregruppen können dabei auch Hydroxylgruppen oder Lewis-saure Zentren die Aktivität und Selektivität beeinflussen. Ebenso kann unabhängig von den Promotoren der Zusatz von Verbindungen eines oder mehrerer der Elemente zu einem Wolframverbindungen enthaltenden Katalysator, ausgewählt aus der Gruppe enthaltend Silizium, Phosphor, Niob, Zink, Zinn, Magnesium, Aluminium oder Molybdän die Oberfläche des Katalysators modifizieren oder die Konzentration der aktiven Zentren verringern, so dass die Selektivität weiter verbessert wird. Speziell die Bildung von fest adsorbierten Hochsiedern oder Koksvorläufern, die aus zwei oder mehreren benachbarten adsorbierten Glycerinmolekülen oder Zwischenverbindungen entstehen, wird dadurch verringert.

Diese Verbindungen dienen somit ebenfalls zur Herabsetzung der Konzentration der aktiven Zentren an der Katalysatoroberfläche und somit zur Erhöhung des Abstandes zwischen benachbarten aktiven Zentren (site isolation). Dies wiederum verringert die Wahrscheinlichkeit, dass zwei Glycerinmoleküle oder gebildete reaktive Zwischenstufen bzw. Koksvorläufer an der Oberfläche miteinander zu höheren Kohlenwasserstoffen reagieren.

Als Festkörperkatalysatoren sind insbesondere auch die aus der US 5,387,720 (EP 0 598 229 A1) bekannten Typen geeignet, wenn sie zusätzlich eine Wolframverbindung und einen oder mehrere der genannten Promotoren enthalten. Dabei handelt es sich um feste, im Reaktionsmedium im Wesentlichen unlösliche ein- oder mehrphasig aufgebaute Stoffe mit einem Hₒ-Wert kleiner +2, vorzugs-weise kleiner -3. Der Hₒ-Wert entspricht der Säurefunktion nach Hammett und läßt sich durch die sogenannte Amintitration unter Verwendung von Indikationen oder durch Adsorption einer gasförmigen Base ermitteln - siehe Studies in surface science and catalysis, Vol. 51, 1989: "New solid acids and bases, their catalytic properties" by K. Tanabe et al. Kapitel 2, insbesondere Seiten 5-9. Kapitel 1 (Seiten 1-3) des vorgenannten Dokuments nennt zahlreiche feste Säuren, aus welchen der Fachmann, gegebenenfalls nach Bestimmung des Hₒ-Wertes, den geeigneten Katalysator für die erfindungsgemäße Modifizierung auswählen kann. Als Basis für die erfindungsgemäßen Dehydratisierungskatalysatoren eignen sich vorzugsweise (i) natürliche und synthetische silikatische Stoffe, wie insbesondere Mordenit, saure Zeolithe und Aktivkohle; (ii) mit ein-, zwei oder mehrbasigen anorganischen Säuren oder sauren Salzen anorganischer Säuren belegte Trägerstoffe wie oxidische oder silikatische Stoffe, beispielsweise Al₂O₃, SiO₂, ZrO₂, TiO₂; Nb₂O₅; (iii) Oxide und Mischoxide, wie beispielsweise γ-Al₂O₃ und ZnO-Al₂O₃-, SiO₂-Al₂O₃-, ZrO₂-SiO₂, 2rO₂-HfO₂-Mischoxide oder Heteropolysäuren.

Geeignete Verbindungen zur Bereitstellung der aktiven Wolfram-Zentren können beispielsweise Ammoniumwolframat, Ammoniummetawolframat, Wolframsäure, Wolframkieselsäure, Wolframphosphorsäure, Wolframoxide oder Heteropolysäuren mit Wolfram als Bestandteil sein. Diese Verbindungen oder deren Mischungen werden dann entweder direkt als Katalysator eingesetzt oder als Katalysatorvorstufen verwendet. Bei Zusatz von weiteren Elementen erfolgt bevorzugt eine vorherige Mischung als Pulver, in einer Lösung oder in einer Schmelze, In einer Ausführungsform der Erfindung werden die katalytisch aktiven Verbindungen auf einem Träger gebunden.

Als Trägermaterialien, können beispielsweise Aluminiumoxid, Titandioxid, Siliziumdioxid, Zirkoniumdioxid, Aktivkohle oder deren Mischungen verwendet werden. Die Träger dienen vorwiegend zur Erhöhung der spezifischen Oberfläche oder zur Fixierung der aktiven Zentren.

Die Herstellung der erfindungsgemäß verwendeten Katalysatoren erfolgt nach für den Fachmann bekannten Verfahren. Werden die aktiven Komponenten auf einen Träger aufgebracht, so erfolgt dies bevorzugt durch Imprägnieren des Trägers, wie z.B. mittels der incipient-wetness Methode durch Besprühen. Die aktiven Komponenten können auch durch Fällen oder Extraktion aus einer Lösung gewonnen werden. Anschließend kann dann eine Formgebung des Katalysators ggf. unter Zugabe von Trägern, Haftvermittlern oder Porenbildnern durch Pressen, Extrudieren, Beschichten oder Agglomerieren erfolgen. Der Katalysator weist üblicherweise einen Partikeldurchmesser zwischen 0,04 mm und 20 mm, bevorzugt zwischen 0,1 und 10 mm, insbesondere zwischen 0,5 und 7 mm auf. Die aktiven Verbindungen können auch in Form einer Schale aufgebracht sein. Wird kein Träger eingesetzt, so ist eine Katalysatorherstellung durch Extrudieren, Pressen von Tabletten oder eine Aufbauagglomeration bevorzugt.

Für die Dehydratisierung in der Gasphase werden Katalysatoren mit einem Hₒ-Wert zwischen -3 und -8,2 besonders bevorzugt. Geeignete Katalysatorsysteme, die Wolframverbindungen und Promotoren enthalten, sind beispielsweise Pd/H₂WO₄ und Pd/WOₓ/ZrO₂.

Gegenstand der Erfindung ist daher ein Verfahren zur Herstellung von Acrolein durch Dehydratisieren von Glycerin in Gegenwart von Wolframverbindungen enthaltenden Festkörperkatalysatoren mit einer Hammett-Acidität H_{O} von <+2 bis bevorzugt -20, die als Promotor Palladium enthalten.

Die Dehydratisierung erfolgt bevorzugt in Abwesenheit von Sauerstoff. In einer Ausführungsform auch in Gegenwart von Wasserstoff in einer Menge von 0,1 bis 10 Vol.%, insbesondere von 0,5 bis 5 %.

Die Dehydratisierung wird in Gegenwart der oben beschriebenen Katalysatoren durchgeführt. Bevorzugt weist die im erfindungsgemäßen Verfahren eingesetzten Katalysatoren eine Hammett-Acidität H₀ von <+2 bis -20 auf. In einer Ausführungsform werden im erfindungsgemäßen Verfahren Katalysatoren eingesetzt, die als Wolfram enthaltenden Bestandteil eine oder mehrere Verbindungen ausgewählt aus der Gruppe Ammoniumwolframat, Wolframphosphorsäure, Wolframsäure, Wolframkieselsäure, Wolframoxide oder Heteropolysäuren mit Wolfram als Bestandteil enthalten.

In einer weiteren Ausführungsform werden im erfindungsgemäßen Verfahren Katalysatoren eingesetzt, die natürliche oder synthetische silikatische oder oxidische Verbindungen enthalten.

In noch einer weiteren Ausführungsform werden im erfindungsgemäßen Verfahren Katalysatoren eingesetzt, die mit ein-, zwei- oder mehrbasigen anorganischen Säuren oder Salzen anorganischer Säuren belegte Trägermaterialien enthalten.

In einer ergänzenden Ausführungsform werden im erfindungsgemäßen Verfahren Katalysatoren eingesetzt, die als Trägermaterialien Aluminiumoxid, Titandioxid, Nb₂O₃, Silizium-dioxid, Aktivkohle oder deren Mischungen oder Mischoxide enthalten.

Die Konzentration des Glycerins im Reaktionsgemisch wird bevorzugt durch die Beimischung von unter den gewählten Reaktionsbedingungen inerten geeigneten gasförmigen Verbindungen gesenkt.

Dadurch werden Nebenreaktionen zu Oligomeren, Polymeren und anderen Hochsiedern minimiert. Eingesetzt werden dem Fachmann bekannte Lösungs- und Verdünnungsmittel wie beispielsweise Wasser, Stickstoff, Luft, Kohlendioxid, Methan und/ oder Wasserstoff, Alkohole wie zum Beispiel Methanol und Ethanol, Aceton, Toluol oder Methylisobutylketon. Bevorzugt werden Verdünnungsmedien, die nach der Kondensation durch Phasentrennung einfach von Acrolein isoliert werden können.

Im erfindungsgemäßen Verfahren wird Glycerin in einer Menge zwischen 1 und 100 %, bevorzugt zwischen 1 und 70 % und insbesondere zwischen 5 und 40 %, bezogen auf die Gesamtmenge des Reaktionsgemisches eingesetzt, wobei die zu 100% fehlende Menge aus unter Reaktionsbedingungen inerten Verbindungen besteht. Bevorzugt wird Stickstoff und/oder Wasser als inerte Verbindung eingesetzt.

Ein Vorteil des Verfahrens besteht darin, dass auch Glycerinlösungen mit einem Gehalt von 5 bis 40 Gew.-% verwendbar sind. Somit sind sogenannte Rohglycerine ohne vorherige Aufkonzentrierung oder Reinigung direkt für die Synthese von Acrolein einsetzbar.

Das erfindungsgemäße Verfahren wird bei einer Temperatur zwischen 150 und 450 °C, bevorzugt zwischen 180 und 350 °C, besonders bevorzugt zwischen 220 und 320 °C durchgeführt. Ferner beträgt der Druck im erfindungsgemäßen Verfahren zwischen 0,1 und 200 bar, bevorzugt zwischen 0,5 und 50 bar, besonders bevorzugt zwischen 0,9 und 10 bar.

Das Verfahren lässt sich in der Flüssigphase oder in der Gasphase durchführen. In beiden Ausführungsformen können im Prinzip die gleichen sauren Feststoffkatalysatoren eingesetzt werden; es hat sich aber gezeigt, dass bestimmte Katalysatoren vorzugsweise für die Dehydratisierung in der Gasphase und andere, vorzugsweise für jene in der Flüssigphase, geeignet sind.

Die Umsetzung in der Gasphase wird besonders bevorzugt, weil der Glycerinumsatz praktisch vollständig ist (> 95 %) und das den Katalysator verlassende gasförmige Reaktionsgemisch unter Erhalt einer wässrigen Acroleinlösung, welche zusätzlich gebildete Nebenprodukte enthält, unmittelbar kondensiert oder absorbiert werden kann; dieses Kondensat lässt sich vielfach unmittelbar weiterverarbeiten. Die partielle Kondensation und/oder Absorption des Reaktionsgemisches kann in mehreren Stufen erfolgen. Sofern erwünscht, kann aus dem Reaktionsgemisch Acrolein, gegebenenfalls gemeinsam mit einem Teil des Wassers, durch fraktionierte Kondensation, Absorption, Desorption und abschließende Destillation gewonnen werden.

In einer Ausführungsform des erfindungsgemäßen Verfahrens wird das Reaktionsgemisch unter Nutzung von partieller Kondensation, Absorption, Desorption und/oder Destillation aufgearbeitet.

Ein Teil des Wassers wird im Kreislauf geführt, wobei es unter Nutzung der Wärmeintegration verdampft und kondensiert wird. Zudem wird ein Inertgas oder ein Verdünnungsmittel im Kreislauf geführt.

Gebildetes Acrolein wird allein oder zusammen mit einem Teil des Wassers und geringen Mengen an Nebenprodukten in bekannter Weise, üblicherweise destillativ, durch Desorption oder durch eine N₂-Strippung aus dem Reaktionsgemisch abgetrennt. Das Acrolein kann durch Kondensation oder eine Wäsche mit Wasser isoliert werden. Das vom Acrolein befreite glycerinhaltige Reaktionsgemisch wird in die Dehydratisierungsstufe zurückgeführt. Ein Vorteil der Dehydratisierung in der Flüssigphase gegenüber jener in der Gasphase besteht in dem geringeren Energieaufwand, weil nur das aus dem Reaktionsgemisch abgetrennte Acrolein sowie ein damit übergehender Teil Wasser verdampft werden müssen.

Die Dehydratisierung in der Gasphase erfolgt vorzugsweise im Temperaturbereich zwischen 240 und 320°C, jene in der Flüssigphase vorzugsweise zwischen 200 und 300°C. Im Falle der Flüssigphasendehydratisierung wird die Apparatur mit mindestens einem solchen Druck beaufschlagt, der zur Aufrechterhaltung der Flüssigphase erforderlich ist.

Die Dehydratisierung erfolgt in einem Festbettreaktor, einem Wirbelschichtreaktor, in einem Reaktor mit einer zirkulierenden Wirbelschicht, einem Wanderbettreaktor oder einem Reaktor mit Regenerator-Riser(-Downer)-Konzept. Sie kann kontinuierlich oder diskontinuierlich durchgeführt werden.

Weiterhin ist die Kombination der Reaktion mit einer Eduktaufbereitung bzw. Produktaufarbeitung mittels einer Reaktivdestillation möglich bzw. sinnvoll, da die Siedepunktunterschiede zwischen Glycerin und Acrolein sehr groß sind. Der Katalysator ist dabei entweder im Sumpf und/oder im Kolonnenteil platziert. Der eingebrachte Katalysator kann beispielsweise in Form einer Schüttung, Suspension oder einer Beschichtung vorliegen. Ein weiterer Vorteil der Reaktivdestillation besteht darin, dass schwersiedende Verunreinigungen aus Roh-Glycerin am Sumpf der Kolonne mit weiteren Schwersiedern, die als Nebenprodukte entstehen können, ausgetragen werden. Acrolein und Leichtsieder werden dann über Kopf abgezogen.

Gebildetes Acrolein kann auch in bekannter Weise allein oder zusammen mit einem Teil des Lösungs- oder Verdünnungsmediums durch Strippen, Destillation oder Extraktion aus dem Reaktionsgemisch abgetrennt werden. Nicht umgesetztes Glycerin kann dann in die Reaktionsstufe zurückgeführt werden.

Der im erfindungsgemäßen Verfahren eingesetzte Katalysator zeichnet sich durch eine gute Regenerierbarkeit und eine langsame Desaktivierung/Verkokung aus.

Die Regeneration kann unter Hydrierungsbedingungen erfolgen. Dabei wird der Koks, der sich während der Reaktion auf der Oberfläche des Katalysators durch Ablagerung von Kohlenwasserstoffen gebildet hat, teilweise oder vollständig entfernt. Bei Palladium als Promotor wird die Regeneration unter hydrierenden Bedingungen durchgeführt.

Die Regeneration erfolgt entweder zeitlich oder örtlich getrennt von der Umsetzung von Glycerin. Bei der zeitlichen Trennung wird die Einspeisung von Glycerin in diesen Reaktor gestoppt und daraufhin die Regeneration durchgeführt, bevor wieder das Eduktgemisch eingespeist wird. Dieser Vorgang wird dann beliebig oft periodisch wiederholt. Für die Durchführung dieser Regenerationsmethode eignet sich insbesondere die Taktung von 2 oder mehreren Festbettreaktoren, um einen kontinuierlichen Produktstrom erzielen zu können. Dabei wird einer der Reaktoren regeneriert, während mindestens einer der Reaktoren zur Produktion von Acrolein eingesetzt wird. Die Zeitintervalle für Reaktion und Regeneration können dabei beliebig gewählt werden. Bevorzugt erfolgt die ununterbrochene Produktion von Acrolein innerhalb eines Zeitintervalls von 2 bis 3000 h, insbesondere 4 bis 400 h bevor der Katalysator in einem Zeitintervall von 0,5 bis 100 h, insbesondere 1 bis 10 h regeneriert wird.

Erfolgt die Regeneration örtlich getrennt, so wird der Katalysator zwischen bevorzugt 2 Reaktoren kontinuierlich bewegt. In einem der Reaktoren findet dabei kontinuierlich die Glycerinumsetzung zu Acrolein statt. Im anderen Reaktor wird der Katalysator kontinuierlich regeneriert. Geeignete Reaktorkonzepte sind dabei der Wanderbettreaktor oder das Regenerator-Riser(-Downer)-Konzept. Das Wanderbett zeichnet sich durch eine geringeren Durchsatz des Katalysators und weniger Katalysatorabrieb aus und ist hier bevorzugt.

Zwischen der Regeneration und der Reaktion ist es jeweils sinnvoll, einen Spülschritt, bevorzugt mit Stickstoff, durchzuführen. Bei der Regeneration werden höhere Temperaturen von 100 bis 800 °C, bevorzugt 200 bis 700 °C, insbesondere 300 bis 550 °C angewandt. Diese müssen nicht mit der Reaktortemperatur während der Glycerinumsetzung übereinstimmen. Dann sind entsprechende Heiz- und Kühlschritte erforderlich. Bevorzugt wird zur Regeneration des Katalysators eine höhere Temperatur als bei der Reaktion angewandt. Der Druck bei der Regeneration beträgt bevorzugt zwischen 0 und 50 bar, insbesondere zwischen 0 und 3 bar.

Zur Regeneration des Katalysators wird Wasserstoff verwendet. Zur Vermeidung hoher Übertemperaturen in der Katalysatorzone durch die exotherme Entfernung des Kokses wird das reduzierende Gas bevorzugt verdünnt eingesetzt, wozu beispielsweise Stickstoff oder Wasserdampf verwendet werden. Während der Regeneration des Katalysators wird die Konzentration des Zusatzstoffes bevorzugt schrittweise erhöht. Der Katalysator kann durch festes Inertmaterial verdünnt werden oder auch in verschiedenen Zonen angeordnet sein.

Die gewünschten katalytischen Eigenschaften und/oder die Säurefunktion des Katalysators verschwindet bei der Regeneration der erfindungsgemäßen Katalysatoren nicht, wie es beispielsweise bei den unter Verwendung von klassischen Säuren wie Phosphorsäure oder Salzsäure hergestellten Katalysatoren beobachtet wird und zur Katalysatordesaktivierung führt.

### Beispiele

### Vergleichsbeispiel 1:

Eingesetzt wurde ein Katalysator gemäß Patentschrift DE 4238493: 100 g Siliziumoxidträger mit einem Durchmesser von etwa 4 mm wurden mit 25 g einer 20 gew.-%igen Phosphorsäure 1 h gemischt. Am Rotationsverdampfer wurde dann bei ca. 70 °C das überschüssige Wasser abgezogen. 18 ml dieses Katalysators wurden in einen Festbettreaktor mit einem Durchmesser von 15 mm gefüllt. Der Reaktor wurde dann auf eine Temperatur von 250 °C beheizt. Mittels einer Pumpe wurden 12,5 g/h einer 20 gew.-%igen wässrigen Glycerinlösung über einen auf 260 °C beheizten Verdampfer in den Reaktor geleitet. Mittels Gaschromatographie wurde der Strom am Reaktorausgang analysiert. Bis zu einer Betriebszeit von etwa 15 h konnte dabei ein vollständiger Umsatz an Glycerin beobachtet werden. Die Selektivität und somit die Ausbeute betrug dabei 79 %. Nach ca. 15 h fiel der Umsatzgrad und somit die Ausbeute jedoch steil ab, so dass nach 23 h nur noch ein Umsatzgrad von 20 % vorlag. Nachdem der Katalysator für 5 h bei einer Temperatur von 350 °C ausschließlich mit einem Wasserstoffstrom von 4 Nl/h durchströmt wurde, konnte keine Verbesserung der Ausbeute (Regenerierung) festgestellt werden. Nachdem der Katalysator für 5 h bei einer Temperatur von 350 °C ausschließlich mit einem Luftstrom von 4 Nl/h durchströmt wurde, konnte sogar eine weitere Verschlechterung der Ausbeute festgestellt werden.

### Vergleichsbeispiel 2:

Vergleichsbeispiel 1 wurde wiederholt, wobei jedoch zu Tabletten gepresste Molybdänsäure als Katalysator eingesetzt wurde. Bei einer Reaktortemperatur von 250 °C konnte innerhalb der ersten 5 h eine Ausbeute von 9 % erreicht werden. Auf eine Regenerierung wurde verzichtet.

### Vergleichsbeispiel 3:

Vergleichsbeispiel 1 wurde wiederholt, wobei jedoch zu Tabletten gepresste Wolframsäure als Katalysator eingesetzt wurde. Bei einer Reaktortemperatur von 260 °C konnte innerhalb der ersten 5 h ein vollständiger Umsatzgrad und eine Ausbeute von 79 % erreicht werden. Innerhalb der nächste Betriebsstunden verringerte sich der Umsatzgrad und entsprechend die Ausbeute deutlich. Im weiteren Verlauf war ein Abfall der Ausbeute von um ca. 5 % pro 10 h festzustellen. Nachdem der Katalysator für 10 h bei einer Temperatur von 350 °C ausschließlich mit einem Wasserstoffstrom von 4 Nl/h durchströmt wurde, konnte die Aktivität des Katalysators deutlich verbessert werden. Der Glycerinumsatz war daraufhin zu Beginn erneut vollständig. Im weiteren Verlauf verringerte sich der Umsatzgrad und die Ausbeute wie vor der Regenerierung beschrieben. Diese Taktung von Glycerindehydratisierung und Regeneration des Katalysators wurde innerhalb von 300 h dreimal wiederholt. Nach Ausbau des unregenerierten Katalysators war dieser schwarz gefärbt. Der Kohlenstoffgehalt des Katalysators betrug 22 Gew.-%, was auf eine erhebliche Verkokung hindeutet.

### Beispiel 1:

Vergleichsbeispiel 1 wurde wiederholt, wobei jedoch zu Tabletten gepresste Wolframsäure als Katalysator eingesetzt wurde. Dieser Katalysator wurde zusätzlich mit 1 Gew.-% Pd imprägniert. Dazu wurde mittels incipient-wetness Pd-Acetat eingesetzt. Bei einer Reaktortemperatur von 260 °C konnte innerhalb der ersten 5 h ein vollständiger Umsatzgrad und eine Ausbeute von 77 % erreicht werden. Innerhalb der nächste Betriebsstunden verringerte sich der Umsatzgrad und entsprechend die Ausbeute deutlich. Nachdem der Katalysator für 10 h bei einer Temperatur von 350 °C ausschließlich mit einem Wasserstoffstrom von 4 Nl/h durchströmt wurde, konnte die Aktivität des Katalysators deutlich verbessert werden. Der Glycerinumsatz war daraufhin zu Beginn erneut vollständig. Im Vergleich zu Beispiel 1 war der Abfall des Umsatzgrades bei der Dehyratisierungsreaktion nach der Regeneration deutlich geringer und das hohe Umsatzgradniveau konnte länger eingehalten werden.

## Patentansprüche

1. Verfahren zur Herstellung von Acrolein durch Dehydratisierung von Glycerin in Gegenwart von Wolframverbindungen enthaltenden Festkörperkatalysatoren mit einer Hammett-Acidität Hₒ von < +2, die als Promotor Palladium enthalten.

2. Verfahren gemäß Anspruch 1, bei dem die Dehydratisierung in Abwesenheit von Sauerstoff erfolgt.

3. Verfahren gemäß den Ansprüchen 1 bis 2, bei dem man Katalysatoren einsetzt, die eine Hammett-Acidität H₀ von < +2 bis -20 aufweisen.

4. Verfahren gemäß den Ansprüchen 1 bis 2, bei dem man Katalysatoren einsetzt, die als Wolfram enthaltenden Bestandteil eine oder mehrere Verbindungen enthalten, ausgewählt aus der Gruppe Ammoniumwolframat, Wolframphosphorsäure, Wolframsäure, Wolframkieselsäure, Wolframoxide oder Heteropolysäuren mit Wolfram als Bestandteil.

5. Verfahren gemäß den Ansprüchen 1 bis 2, bei dem man Katalysatoren einsetzt, die natürliche oder synthetische silikatische oder oxidische Verbindungen enthalten.

6. Verfahren gemäß den Ansprüchen 1 bis 2, bei dem man Katalysatoren einsetzt, die mit ein-, zwei- oder mehrbasigen anorganischen Säuren oder Salzen anorganischer Säuren belegte Trägermaterialien enthalten.

7. Verfahren gemäß den Ansprüchen 1 bis 2, bei dem man Katalysatoren einsetzt, die als Trägermaterialien Aluminiumoxid, Titandioxid, Nb₂O₃, Siliziumdioxid, Zirkoniumoxid, Aktivkohle oder deren Mischungen oder Mischoxide enthalten.

8. Verfahren gemäß den Ansprüchen 1 bis 7, bei dem die Dehydratisierung in Anwesenheit von Wasserstoff erfolgt.

9. Verfahren gemäß den Ansprüchen 1 bis 7, bei dem man Glycerin in einer Menge von 1 bis 100% bezogen auf die Gesamtmenge des Reaktionsgemisches einsetzt, wobei die zu 100% fehlende Menge aus unter Reaktionsbedingungen inerten Verbindungen besteht.

10. Verfahren gemäß Anspruch 9, bei denen man als inerte Verbindung Stickstoff und/oder Wasser einsetzt.

11. Verfahren gemäß den Ansprüchen 1 bis 10, **dadurch gekennzeichnet, dass** dies bei Drücken zwischen 0,1 und 200 bar sowie bei Temperaturen zwischen 150 °C und 450 °C erfolgt.

12. Verfahren gemäß den Ansprüchen 1 bis 11, bei dem die Umsetzung in der Gasphase erfolgt.

13. Verfahren gemäß den Ansprüchen 1 bis 12, bei dem man die Dehydratisierung in einem Festbettreaktor, einem Wirbelschichtreaktor, in einem Reaktor mit einer zirkulierenden Wirbelschicht, einem Wanderbettreaktor oder einem Reaktor mit Regenerator-Riser(-Downer)-Konzept durchführt.

14. Verfahren gemäß den Ansprüchen 1 bis 13, bei dem das Reaktionsgemisch unter Nutzung von partieller Kondensation, Absorption, Desorption, und/oder Destillation aufgearbeitet wird.

15. Verfahren gemäß den Ansprüchen 1 bis 14, bei dem ein Teil des Wassers im Kreislauf geführt und unter Nutzung der Wärmeintegration verdampft und kondensiert wird.

16. Verfahren gemäß den Ansprüchen 1 bis 15, bei dem ein Inertgas oder Verdünnungsmittel im Kreislauf geführt wird.

## Claims

1. Process for preparing acrolein by dehydrating glycerol in the presence of solid-state catalysts which comprise tungsten compounds and have a Hammett acidity HO of < +2 and which comprise palladium as promoter.

2. Process according to Claim 1, in which the dehydration is effected in the absence of oxygen.

3. Process according to Claims 1 to 2, in which catalysts having a Hammett acidity Hₒ of < +2 to - 20 are used.

4. Process according to Claims 1 to 2, in which catalysts comprising, as the tungsten-containing constituent, one or more compounds selected from the group of ammonium tungstate, tungstophosphoric acid, tungstic acid, tungstosilic acid, tungsten oxides or heteropolyacids with tungsten as a constituent are used.

5. Process according to Claims 1 to 2, in which catalysts comprising natural or synthetic, silicatic or oxidic compounds are used.

6. Process according to Claims 1 to 2, in which catalysts comprising support materials coated with mono-, di- or polybasic inorganic acids or salts of inorganic acids are used.

7. Process according to Claims 1 to 2, in which catalysts comprising, as support material, aluminium oxide, titanium dioxide, Nb₂O₃, silicon dioxide, zirconium oxide, activated carbon or the mixtures or mixed oxides thereof are used.

8. Process according to Claims 1 to 7, in which the dehydration is effected in the presence of hydrogen.

9. Process according to Claims 1 to 7, in which glycerol is used in an amount of 1 to 100% based on the total amount of the reaction mixture, the remaining amount up to 100% consisting of compounds inert under reaction conditions.

10. Process according to Claim 9, in which the inert compound used is nitrogen and/or water.

11. Process according to Claims 1 to 10, **characterized in that** it is effected at pressures between 0.1 and 200 bar and at temperatures between 150 and 450°C.

12. Process according to Claims 1 to 11, in which the reaction is effected in the gas phase.

13. Process according to Claims 1 to 12, in which the dehydration is performed in a fixed bed reactor, a fluidized bed reactor, in a reactor with a circulating fluidized bed, a moving bed reactor or a reactor with regenerator-riser (-downer) design.

14. Process according to Claims 1 to 13, in which the reaction mixture is worked up with utilization of partial condensation, absorption, desorption and/or distillation.

15. Process according to Claims 1 to 14, in which a portion of the water is circulated and is evaporated and condensed with utilization of thermal integration.

16. Process according to Claims 1 to 15, in which an inert gas or diluent is circulated.

## Revendications

1. Procédé de fabrication d'acroléine par déshydratation de glycérine en présence de catalyseurs solides contenant des composés de tungstène ayant une acidité Hammett H₀ < +2, qui contiennent du palladium en tant que promoteur.

2. Procédé selon la revendication 1, dans lequel la déshydratation a lieu en l'absence d'oxygène.

3. Procédé selon les revendications 1 à 2, dans lequel des catalyseurs qui présentent une acidité Hammett H₀ de < +2 à -20 sont utilisés.

4. Procédé selon les revendications 1 à 2, dans lequel des catalyseurs qui contiennent en tant que constituant contenant du tungstène un ou plusieurs composés choisis dans le groupe constitué par le tungstate d'ammonium, l'acide tungstène-phosphorique, l'acide tungstique, l'acide tungstène-silicique, les oxydes de tungstène ou les hétéropolyacides contenant du tungstène en tant que constituant sont utilisés.

5. Procédé selon les revendications 1 à 2, dans lequel des catalyseurs qui contiennent des composés silicatiques ou oxydiques naturels ou synthétiques sont utilisés.

6. Procédé selon les revendications 1 à 2, dans lequel des catalyseurs qui contiennent des matériaux supports revêtus avec des acides inorganiques mono-, bi- ou polybasiques ou des sels d'acides inorganiques sont utilisés.

7. Procédé selon les revendications 1 à 2, dans lequel des catalyseurs qui contiennent en tant que matériaux supports de l'oxyde d'aluminium, du dioxyde de titane, Nb₂O₃, du dioxyde de silicium, de l'oxyde de zirconium, du charbon actif ou leurs mélanges ou oxydes mixtes sont utilisés.

8. Procédé selon les revendications 1 à 7, dans lequel la déshydratation a lieu en présence d'hydrogène.

9. Procédé selon les revendications 1 à 7, dans lequel la glycérine est utilisée en une quantité de 1 à 100 %, par rapport à la quantité totale du mélange réactionnel, la quantité manquante pour atteindre 100 % étant constituée par des composés inertes dans les conditions de réaction.

10. Procédé selon la revendication 9, dans lequel de l'azote et/ou de l'eau sont utilisés en tant que composé inerte.

11. Procédé selon les revendications 1 à 10, dans lequel celui-ci a lieu à des pressions comprises entre 0,1 et 200 bar, et à des températures comprises entre 150 °C et 450 °C.

12. Procédé selon les revendications 1 à 11, dans lequel la réaction a lieu en phase gazeuse.

13. Procédé selon les revendications 1 à 12, dans lequel la déshydratation est réalisée dans un réacteur à lit fixe, un réacteur à lit fluidisé, dans un réacteur à lit fluidisé en circulation, un réacteur à lit mobile ou un réacteur à concept régénérateur-colonne montante (descendante).

14. Procédé selon les revendications 1 à 13, dans lequel le mélange réactionnel est traité en utilisant une condensation partielle, une absorption, une désorption et/ou une distillation.

15. Procédé selon les revendications 1 à 14, dans lequel une partie de l'eau est mise en circulation, et évaporée et condensée en utilisant l'intégration de la chaleur.

16. Procédé selon les revendications 1 à 15, dans lequel un gaz inerte ou un diluant est mis en circulation.
